# EUROPÄISCHE PATENTSCHRIFT

(11) **EP 1 928 373 B1**
(45) Veröffentlichungstag und Bekanntmachung des Hinweises auf die Patenterteilung: **09.12.2009**
(21) Anmeldenummer: 06775175.0
(22) Anmeldetag: 08.09.2006
(51) Int. Cl.: A61F 5/058

(54) **Mallet-Fingerschiene**
Mallet finger splint
Attelle pour doigt en maillet

(30) Priorität: 16.09.2005 CH 15152005
(43) Veröffentlichungstag der Anmeldung: 11.06.2008
(73) Patentinhaber: Chrisofix AG, 8212 Neuhausen am Rheinfall (CH)
(72) Erfinder: BOLLA, Kalman, CH-8212 Neuhausen am Rheinfall (CH)
(74) Vertreter: Rentsch & Partner
(86) Internationale Anmeldenummer: PCT/CH2006/000482
(87) Internationale Veröffentlichungsnummer: WO 2007/030962

(56) Entgegenhaltungen:
- US-A- 4 798 199
- US-A- 5 230 699
- US-B1- 6 575 925
- US-B2- 6 692 452

## Beschreibung

### TECHNISCHES GEBIET

Die vorliegende Erfindung bezieht sich auf das Gebiet der medizinischen Schienen. Sie betrifft eine Mallet-Fingerschiene gemäss dem Oberbegriff des Anspruchs 1.

### STAND DER TECHNIK

Unter einem Mallet-Finger oder "dropped" Finger oder Baseball-Finger ist eine Fingerverletzung zu verstehen, bei welcher der Mechanismus, der den Finger an seinem vorderen Ende (am ersten (distalen) Fingergelenk zwischen dem ersten Fingerglied und dem mittleren Fingerglied) streckt, unterbrochen ist. Diese Art der Fingerverletzung tritt beispielsweise auf, wenn (beim Baseball oder anderen Ballspielen) ein Ball mit Wucht direkt auf die Fingerspitze trifft.

Zur Therapie einer solchen Fingerverletzung werden spezielle Mallet-Fingerschienen eingesetzt, die den verletzten Finger in der gestreckten Stellung mit leicht nach oben gebogenem ersten Fingerglied fixieren. Aus dem Stand der Technik sind verschiedenartige Mallet-Fingerschienen bekannt, die jeweils mit bestimmten Nachteilen verbunden sind.

In der DE-U1-84 32 615 wird eine Mallet-Fingerschiene beschrieben, die aus einer das äusserste Fingergelenk und das erste Fingerglied unterseitig stützenden Schale und einer das äusserste (distale) Fingergelenk und das mittlere Fingerglied oberseitig stützenden Schale bestehen, die rohrartig miteinander verbunden sind, wobei die unterseitige Schale in vorbestimmter Weise ausgeschnitten ist. Derartige rohrförmige Schienen sind formstabil, einfach herstell- und anwendbar und geben dem verletzten Finger einen allseitigen Schutz. Nachteilig ist jedoch, dass diese Schienen steif und nicht anpassbar sind. Es müssen daher für unterschiedlich grosse Finger unterschiedliche Schienengrössen hergestellt und bereit gehalten werden, um durch eine gute Anpassung eine ausreichende Fixierung zu erreichen. Ähnliches gilt auch für die in der DE-A1-35 17 073 offenbarte Fingerschiene.

Aus der GB-929 317 ist eine Mallet-Fingerschiene bekannt, die aus mehreren Blechteilen zusammengesetzt ist, die in verstellbarer Weise miteinander verbunden werden können, um an unterschiedlich lange Finger angepasst zu werden. Hierdurch ist es möglich, mit einem Satz von Teilen unterschiedlich grosse Finger zu schienen. Nachteilig ist jedoch die vergleichsweise aufwändige Herstellung und komplizierte Handhabung, bei der zunächst die Blechteile zusammengesetzt, dann die Polsterung aufgelegt, und schliesslich die Schiene am Finger befestigt werden muss.

In der US-B2-6,692,452 ist eine Mallet-Fingerschiene offenbart, die aus einem über den Finger steckbaren elastischen Schlauch besteht, der auf der Unterseite eine Tasche aufweist, in die eine formgebende steife Platte aus einem geeigneten Kunststoffmaterial eingeschoben werden kann. Auch hier besteht das Problem, dass bei unterschiedlich dicken Fingern entweder der Schlauch zu stramm oder zu locker sitzen kann, oder Schläuche mit unterschiedlichem Durchmesser verwendet werden müssen.

Die US-A-5,925,008 zeigt eine Mallet-Fingerschlene, die aus einem auf der Oberseite des Fingers angeordneten, abgewinkelten Schienenteil besteht, das mit dem verletzten Finger durch um Finger und Schienenteil gewickelte Bandagen verbunde wird. Obgleich der Aufbau einfach und eine Anpassbarkeit an unterschiedliche Fingergrössen gegeben ist, gestaltet sich die Anwendung eher schwierig, weil hier verschiedene separate Bandagen angelegt werden müssen, um durch eine enge Verbindung zwischen dem Schienenteil und dem Finger die gewünschte Streckstellung des Fingers zu erreichen.

Aus der US-A1-2005/0027223 ist schliesslich eine Mallet-Fingerschiene bekannt, die aus drei separaten, durch einen steifen Draht längen- und winkeljustierbar miteinander verbundenen schalenförmigen Segmenten besteht, die den einzelnen Fingergliedern zugeordnet sind. Die Schiene wird mittels Klettbändem am ersten und letzten Fingerglied befestigt. Eine solche Schiene ist aufgrund des komplizierten mechanischen Aufbaus in der Herstellung sehr aufwändig und wirkt sich am Finger störend aus.

In der Druckschrift US-B1-6,575,925 ist eine Fingerschiene mit einer sogenannten "Baseball-Konfiguration" offenbart, die aus einer länglichen Schale aus Aluminium, Kunststoff oder Schaumstoff besteht, auf deren Oberseite eine Auflage angeordnet ist, und die mittels eines Bandes oder mehrerer Bänder mit Klettverschluss befestigt wird, die in sich geschlossen jeweils durch eine Öse auf der Unterseite der Schiene und über den auf der Schiene liegenden Finger geführt werden. Obgleich in der Beschreibung auch eine Löffelform erwähnt wird, hat die Schiene auf der Unterseite des Fingers eine gleichmässig grosse Breite. Darüber hinaus erstreckt sie sich vom distalen Ende des Fingers bis zum proximalen Ende direkt am Übergang zur Handfläche. Hieraus ergibt sich eine Vielzahl von Nachteilen:
- Die über alle Fingerglieder gehende Länge der Schiene beeinträchtigt unnötigerweise erheblich die Bewegungsmöglichkeit des geschienten Fingers. Insbesondere kann der Finger nicht am innersten (proximalen) und am mittleren Gelenk abgebogen werden.
- Die über die gesamte Fingerlänge reichende grosse Breite der Schiene beeinträchtigt auch die Beweglichkeit der benachbarten ungeschienten Finger.
- Die auf der Unterseite der Schiene angebrachten Ösen zum Durchfädeln des Befestigungsbandes sind beim Einsatz der Hand hinderlich, weil sie hinter Gegenstände hinterhaken können. Sie verkomplizieren die Herstellung und Anwendung der Schiene und verhindern, dass die Schiene mit einer Hülle hergestellt werden kann. Des weiteren sind sie hinderlich bei einer Anpassung der Schiene an den Finger.
- Das in sich selbst geschlossene Klettband zur Befestigung hat an einem Ende eine Hakenfläche, die dem Finger zugewandt ist und daher den Tragekomfort beeinträchtigen kann.

Ein weiteres allgemeines Problem bei den bekannten Mallet-Fingerschienen ist, das die Schienen zum Verrutschen neigen, so dass die gewünschte überstreckte Lagerung des verletzten Fingers nur bedingt gewährleistet ist. Es ist daher wünschenswert, bei einer verbesserten Mallet-Fingerschiene auch diesem Missstand abzuhelfen.

Zusammenfassend kann gesagt werden, dass die bekannten Mallet-Fingerschienen die folgenden Nachteile haben:
- Die Schienen erstrecken sich üblicherweise nicht nur über das äussere (distale) Fingergelenk, sondern auch über das mittlere Fingergelenk und reichen meist bis an die Handfläche. Hierdurch wird die Bewegungsmöglichkeit des geschienten Fingers stark eingeschränkt, ohne das dies für die eigentliche Schienung von Nutzen ist.
- Wenn die Schienen bis an die Handfläche reichen, wird durch ein Abwinkeln des geschienten Fingers am inneren (proximalen) Fingergelenk auf das hintere Ende der Schiene eine in Schienenlängsrichtung nach aussen wirkende Kraft ausgeübt, die zu einer Verschiebung der Schiene relativ zum Finger (Vorrutschen) führen kann und die Wirkung der Schiene beinträchtigt.
- Die bekannten Schienen nehmen viel Raum ein, der nicht nur die Beweglichkeit des geschienten Fingers, sondern auch den Bewegungsspielraum der anderen Finger der selben Hand einschränkt.
- Dies gilt auch für die Befestigungsmittel, mit denen die Fingerschiene am Finger befestigt wird. Diese Befestigungsmittel sind häufig voluminös, unbequem und schwer zu handhaben.

### DARSTELLUNG DER ERFINDUNG

Es ist daher Aufgabe der Erfindung, eine Mallet-Fingerschiene anzugeben, welche einfach aufgebaut und herzustellen ist, in der Anwendung schnell und unkompliziert ist, sich durch geringe Abmessungen und geringes Gewicht auszeichnet, und einen hohen Tragekomfort mit möglichst wenig Bewegungseinschränkungen bietet und gegen ein unbeabsichtigtes Verrutschen am Finger abgesichert ist.

Die Aufgabe wird durch die Gesamtheit der Merkmale des Anspruchs 1 gelöst. Der Kern der Erfindung besteht darin, der Mallet-Fingerschiene die Form eines Löffels mit einem kurzen Stiel und einer leicht aufwärts gebogenen Laffe zu geben, wobei die Mallet-Fingerschiene derart ausgebildet ist, dass der Stiel unter dem mittleren Fingerglied liegt, ohne die Bewegung des mittleren Fingergelenks zu beeinträchtigen, während das vordere Fingerglied in der Laffe zu liegen kommt. Die Laffe bietet eine Mulde, in die das erste Fingerglied in Streckstellung des vorderen Fingergelenks sicher und bequem eingelegt werden kann, während der Stiel im Zusammenwirken mit dem mittleren Fingerglied die Fixierung der Schiene am Finger übernimmt. Die Fingerschiene nach der Erfindung kann sehr kurz und leicht ausgeführt werden und lässt sich wegen des Stiels sehr einfach befestigen. Da der Stiel so kurz gehalten ist, dass er die Beweglichkeit des mittleren Fingergelenks nicht beeinträchtigt, wird beim Abknicken des mittleren Fingergelenks keine Längskraft auf die Schiene ausgeübt und ein dadurch bedingtes Verrutschen der Schiene relativ zum Finger sicher vermieden.

Eine Ausgestaltung der Mallet-Fingerschiene nach der Erfindung ist **dadurch gekennzeichnet, dass** zumindest auf der Aussenseite des Stiels Mittel zum lösbaren Befestigen eines Befestigungsbandes vorgesehen sind, welche vorzugsweise Haken nach Art eines Klettverschlusses umfassen. Hierdurch ist es möglich, zur sicheren und bequemen zu tragenden Befestigung der Schiene ein einfaches, insbesondere textiles Befestigungsband zu verwenden, das um den Finger geschlungen wird und mit beiden Enden auf der Aussenseite des Stils lösbar befestigt wird.

Um die Schiene noch effektiver gegen ein Verrutschen zu sichern, kann es von Vorteil sein, wenn an der Mallet-Fingerschiene rutschhemmende Mittel vorgesehen sind, welche eine relative Verschiebung zwischen Mallet-Fingerschiene und Finger hemmen. Auf diese Weise bleibt die Fingerschiene unabhängig von den auf sie von aussen einwirkenden Kräften sicher in ihrer Lage relativ zum Finger.

Insbesondere ist die Mallet-Fingerschiene einstückig ausgebildet.

Gemäss einer bevorzugten Weiterbildung der Erfindung ist die Mallet-Fingerschiene aus einem Blech geformt, wobei vorzugsweise als Blech ein Aluminiumblech verwendet wird.

Damit die Fingerschiene bei ausreichender Steifigkeit in gewissen Grenzen an den Finger anpassbar ist, ist es von Vorteil, wenn das Blech mit der Hand plastisch verformbar ist, und wenn zur Längsversteifung in das Blech Wellen eingeformt sind, deren Kämme in Schienenlängsrichtung bzw. in Längsrichtung des Fingers verlaufen. Zusätzlich kann das Blech zur Verbesserung der Atmungsaktivität perforiert sein.

Zur Erhöhung der Bequemlichkeit und Bereitstellung weiterer Funktion wie z.B. einer verbesserten Rutschhemmung kann das Blech auf der Innenseite und/oder der Aussenseite mit wenigstens einer Schicht bedeckt sein. Insbesondere ist das Blech auf beiden Seiten mit einer Zwischenschicht aus Kunststoffschaum, insbesondere PU-Schaum, bedeckt, und ist auf den Zwischenschichten jeweils eine weitere Deckschicht angeordnet, die zusätzliche Funktionen übernimmt.

Die Zwischenschichten sind vorzugsweise, z.B. mit einem Heisskleber, mit dem Blech verklebt; die Zwischenschichten und die zugehörigen Deckschichten sind jeweils zusammenlaminiert.

Für den Tragekomfort und die einfache Handhabung ist es von Vorteil, wenn die innere Deckschicht aus einem schweissableitenden Textilmaterial besteht und die äussere Deckschicht aus einem für einen Klettverschluss geeigneten Textilmaterial besteht. Die innere Deckschicht kann zusätzlich mit einer die Vermehrung der Schweissbakterien verhindernden Funktion ausgestattet sein.

Zur Befestigung der Mallet-Fingerschiene am Finger ist vorzugsweise ein Befestigungsband vorgesehen, welches mit beiden Enden am Stiel lösbar befestigt ist und das darauf liegende mittlere Fingerglied umschliesst, wobei zur Befestigung des Befestigungsbandes an der Mallet-Fingerschiene insbesondere auf der Aussenseite des Stiels ein Klettverschluss angeordnet ist. Eine solche Befestigung ist extrem platzsparend, leicht zu handhaben und bequem zu tragen, weil sich das Befestigungsband an den Finger anpasst und auf dem Finger weich aufliegt.

Um ein Verrutschen der Fingerschiene entlang dem Finger zu verhindern, ist es günstig, wenn die rutschhemmenden Mittel einen auf die obere Schicht des Stiels aufgeklebten doppelten Klebestreifen umfassen.

Eine weitere Verbesserung ergibt sich, wenn am Befestigungsband zur Fixierung des Befestigungsbandes relativ zum Finger zusätzliche rutschhemmende Mittel angeordnet sind, die vorzugsweise ein doppelseitiges Klebeband umfassen und die, wenn das Befestigungsband um den Finger gewickelt ist, auf der Oberseite des Fingers zu liegen kommen.

Die Mallet-Fingerschiene kann aber auch aus einem Kunststoff geformt sein,
wobei dann vorzugsweise auf der Aussenseite des Stils eine Hakenschicht als Teil eines Klettverschlusses angebracht ist.

Gemäss einer anderen Ausgestaltung umfasst die Mallet-Fingerschiene eine Hülle, die eine Oberlage und eine Unterlage aufweist, wobei der zu schienende Finger auf der Oberlage zu liegen kommt, und die Unterlage die Mallet-Fingerschiene auf der Unterseite abdeckt. Mit Vorteil hat die Oberlage rutschhemmende Eigenschaften, und weist insbesondere Silikon, vorzugsweise in Form von eingebetteten Kügelchen, auf. Die Unterlage ist bevorzugt als Haken enthaltender Teil eines Klettverschlusses ausgebildet.

### KURZE ERLÄUTERUNG DER FIGUREN

Die Erfindung soll nachfolgend anhand von Ausführungsbeispielen im Zusammenhang mit der Zeichnung näher erläutert werden. Es zeigen
- Fig. 1: in einer perspektivischen Seitenansicht ein Ausführungsbeispiel der Mallet-Fingerschlene nach der Erfindung mit einem Befestigungsband;
- Fig. 2: eine an einem Finger angebrachte Mallet-Fingerschiene nach Fig. 1;
- Fig. 3: den Querschnitt durch die am Finger angebrachte Mallet-Fingerschiene gemäss Fig. 2 im Bereich des Stiels bzw. des mittleren Fingergliedes;
- Fig. 4: in einer zu Fig. 3 vergleichbaren Darstellung den Querschnitt durch die am Finger angebrachte Mallet-Fingerschiene gemäss Fig. 2 im Bereich der Laffe bzw. des ersten Fingergliedes;
- Fig. 5: eine vergrösserte schematisierte Darstellung des Querschnitts durch die Mallet-Fingerschiene nach Fig. 1 im Bereich des Stiels;
- Fig. 6: im Querschnitt durch den Stil eine aus Kunststoff geformte Mallet-Fingerschiene gemäss einem anderen Ausführungsbeispiel der Erfindung mit auf der Unterseite des Stils angeformter Hakenschicht, die der Befestigung der beiden Enden eines Befestigungsbandes dient;
- Fig. 7: im Querschnitt durch den Stil eine Maltet-Fingerschiene gemäss einem weiteren Ausführungsbeispiel der Erfindung, die in einer aus einer Oberlage und einer Unterlage bestehenden Hülle untergebracht ist, die zur Rutschsicherung und Befestigung des Befestigungsbandes dient;
- Fig. 8-10: verschiedene Schritte bei der Herstellung einer Mallet-Fingerschiene nach Fig. 7 gemäss einem Ausführungsbeispiel der Erfindung.

### WEGE ZUR AUSFÜHRUNG DER ERFINDUNG

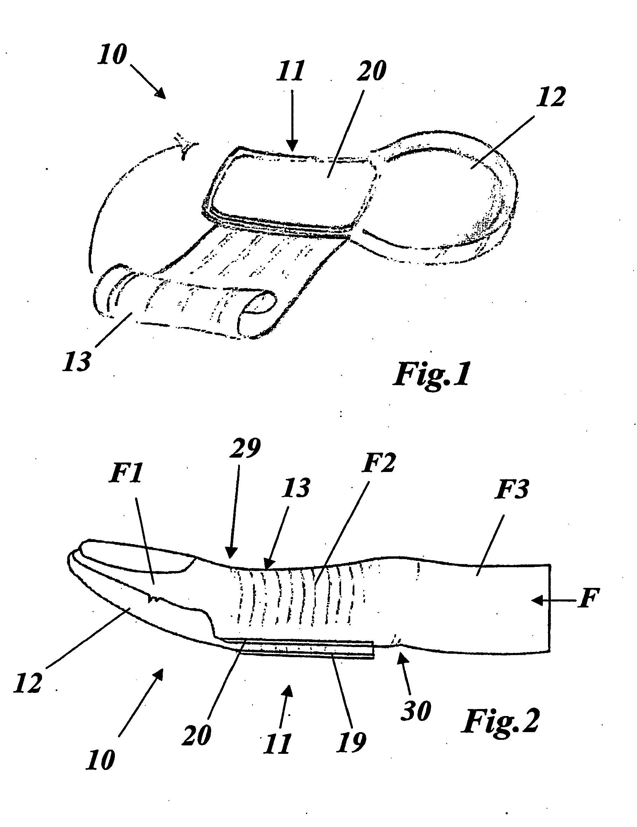

In Fig. 1 ist in einer perspektivischen Seitenansicht ein Ausführungsbeispiel einer Mallet-Fingerschiene nach der Erfindung wiedergegeben. Die Mallet-Fingerschiene 10 hat die generelle Form eines kurzstieligen Löffels mit einem flachen Stiel 11 und einer daran anschliessenden gewölbten Laffe 12. Die Mallet-Fingerschiene 10 mit ihrer Löffelform ist derart ausgebildet, dass gemäss Fig. 2 bei an einem Finger F angebrachter Schiene der Stiel 11 unter dem mittleren (medialen) Fingerglied F2 liegt, während das vordere (distale) Fingerglied F1 in der Laffe 12 zu liegen kommt. Damit das vordere Fingergelenk 29 zwischen dem vorderen (distalen) Fingerglied F1 und dem mittleren (medialen) Fingerglied F2 in einer gestreckten Position gehalten wird, ist die Laffe 12 gegenüber dem Stiel 11 leicht nach oben gebogen. Aufgrund der Biegsamkeit der Schiene kann die Stellung der Laffe 12 relativ zum Stiel 11 jedoch angepasst und verändert werden. Ebenso können die seitlichen Ränder der Laffe 12 stärker hochgebogen werden, um dem geschienten Finger F mehr seitlichen Halt zu geben.

Die Länge des Stiels 11 ist gemäss Fig. 2 der Länge des mittleren (medialen) Fingergliedes F2 angepasst. Die Mallet-Fingerschiene 10 erstreckt sich so nur über die beiden vorderen Fingerglieder F1 und F2. Das hintere (proximale) Fingerglied F3 bleibt dagegen frei. Die Mallet-Fingerschiene 10 ist einstückig ausgebildet und lässt sich so sehr einfach herstellen und anwenden. Grundelement der Mallet-Fingerschiene 10 ist ein Aluminiumblech 14, das sich durch den Stiel 11 und die Laffe 12 hindurch erstreckt. Damit die Mallet-Fingerschiene 10 insbesondere im Bereich der Laffe 12 an den jeweiligen Finger F bzw. die Fingerglieder F1, F2 angepasst werden kann, ist die Dicke des Bleches 14 so gewählt (einige 1/10 Millimeter, vorzugsweise zwischen 0,2 und 0,6 mm), dass das Blech 14 mit der Hand plastisch verformbar ist. Um trotz der (begrenzten) Verformbarkeit eine für die Schienung notwendige Steifigkeit zu erreichen, sind zur Längsversteifung in das Blech 14 Wellen eingeformt, deren Kämme in Schienenlängsrichtung bzw. in Längsrichtung des Fingers F verlaufen. Ein vergleichbares Blech (mit anderer Orientierung der Wellen) ist vom selben Anmelder bereits bei anderen Schienen vorgeschlagen worden (EP-B1-0 874 607).

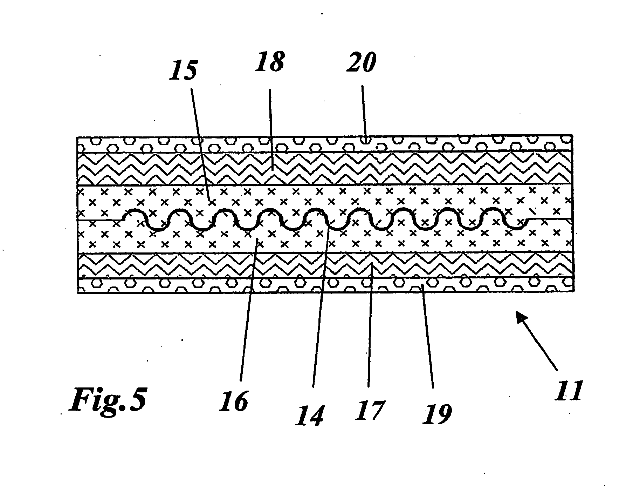

Über dem Blech sind auf der Ober- und Unterseite mehrere Schichten aufgebracht, die verschiedene Funktionen hinsichtlich Tragekomfort und Befestigung der Schiene übernehmen. Damit hat die Mallet-Fingerschiene 10 im Bereich des Stiels 11 den in Fig. 5 dargestellten Aufbau: Das zentrale gewellte Aluminiumblech 14 ist beidseitig mit einer Zwischenschicht 15 bzw. 16 aus PU-Schaum verklebt. Die beiden 1-2 mm dicken Zwischenschichten 15, 16 betten das Blech 14 ein und bilden eine erste Polsterung. Über den Zwischenschichten 15, 16 sind oben und unten Deckschichten 18 bzw. 17 angeordnet, die mit der jeweiligen Zwischenschicht zusammenlaminiert sind.

Die innere Deckschicht 18 besteht vorzugsweise aus einem schweissableitenden Textilmaterial. Sie ist mit Vorteil zusätzlich mit einer die Vermehrung der Schweissbakterien verhindernden Funktion ausgestattet, die durch eine spezielle Materialauswahl oder durch eine Imprägnierung mit entsprechenden Substanzen erreicht werden kann. Dadurch ist gewährleistet, dass der über längere Zeit geschiente Finger F trocken liegt und geruchsneutral bleibt. Darüber hinaus wird durch eine Perforation des Bleches 14 sichergestellt, das Luft an die von der Schiene bedeckte Unterseite des Fingers gelangen kann. Das Textilmaterial der Deckschicht 18 trägt zusätzlich durch seine textile Beschaffenheit zum Tragekomfort der Mallet-Fingerschiene 10 bei.

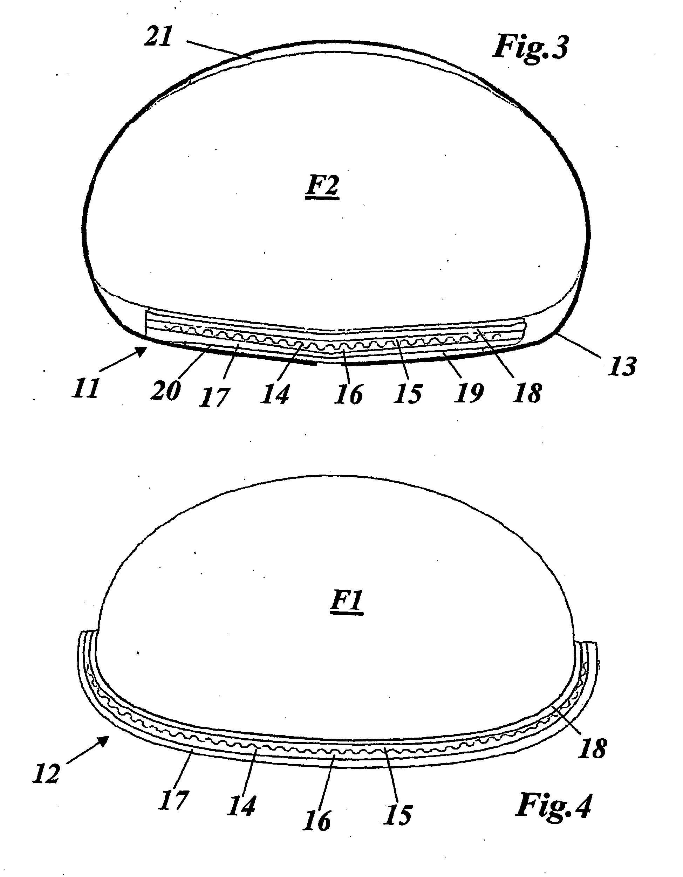

Die äussere Deckschicht 17 besteht aus einem für einen Klettverschluss geeigneten Textilmaterial, das den Charakter von Velour oder Flausch hat und so mit der Hakenseite eines Klettbandes haftend zusammenwirken kann. Zur Befestigung der Mallet-Fingerschlene 10 am Finger F ist ein Befestigungsband 13 vorgesehen, welches fest um den Stiel 11 und das darauf liegende mittlere (mediale) Fingerglied F2 wickelbar ist (Fig. 2, 3). Das Befestigungsband 13 ist gemäss Fig. 3 mit beiden Enden an der Mallet-Fingerschiene 10 befestigt. Dazu ist an der Mallet-Fingerschiene 10 auf der Aussen- bzw. Unterseite des Stiels 11 ein Klettverschluss ausgebildet, indem ein doppelseitiges Klettband 19 mit der einen Seite auf der unteren Deckschicht 17 befestigt (eingehakt) ist. Die andere, freie Seite des Klettbandes 19 dient der beidendigen Befestigung des Befestigungsbandes 13 (Fig. 3). Dazu ist das Befestigungsband 13 auf wenigstens einer Seite mit einem geeigneten Velours oder Flausch ausgerüstet, der zugleich den Tragekomfort erhöht.

Wie in Fig. 2 gezeigt ist die Mallet-Fingerschiene 10 mit dem Stiel 11 fest am mittleren (medialen) Fingerglied F2 befestigt, während die nach vorne hervorstehende Laffe 12 das vordere (distale) Fingerglied F1 in leicht überstreckter Haltung stützt und stabilisiert. Der Stiel 11 ist so kurz gehalten, dass das mittlere Fingergelenk 30 gebeugt werden kann, ohne dass das freie Stielende am hinteren Fingerglied F3 anstösst und damit eine Verschiebungskraft auf die Schiene ausübt. Um auch sonst eine Verschiebung der Mallet-Fingerschiene 10 in Fingerlängsrichtung nach vorn und damit ein Nachlassen der Stützfunktion der Laffe 12 sicher zu verhindern, sind auf der Innenseite des Stiels 11 zur Fixierung der Mallet-Fingerschiene 10 relativ zum Finger F zusätzliche Haftmittel in Form eines auf die obere Schicht 18 des Stiels 11 aufgeklebten doppelten Klebestreifens 20 vorgesehen. Der Klebestreifen 20 ist vorzugsweise aus einem Gewebematerial, um die Luft- und Feuchtigkeitsdurchlässigkeit im Schienenbereich nicht zu beeinträchtigen (atmungsaktiv).

Eine zusätzliche Fixierung der Schiene am Finger F kann durch ein weiteres doppeltes Klebeband 21 auf der Oberseite des mittleren (medialen) Fingergliedes F2 erreicht werden, durch welches die Lage des Befestigungsbandes 13 relativ zum Finger stabilisiert wird (Fig. 3). Das doppelte Klebeband 21, das ebenfalls atmungsaktiv sein kann, kann dazu an einem entsprechenden Ort (in einem mittleren Abschnitt) auf dem Befestigungsband 13 angebracht sein.

Es versteht sich von selbst, dass die doppelten Klebebänder oder vergleichbare Haftmittel auch bei der Fixierung anderer Schienen relativ zum geschienten Glied wirkungsvoll einsetzbar sind.

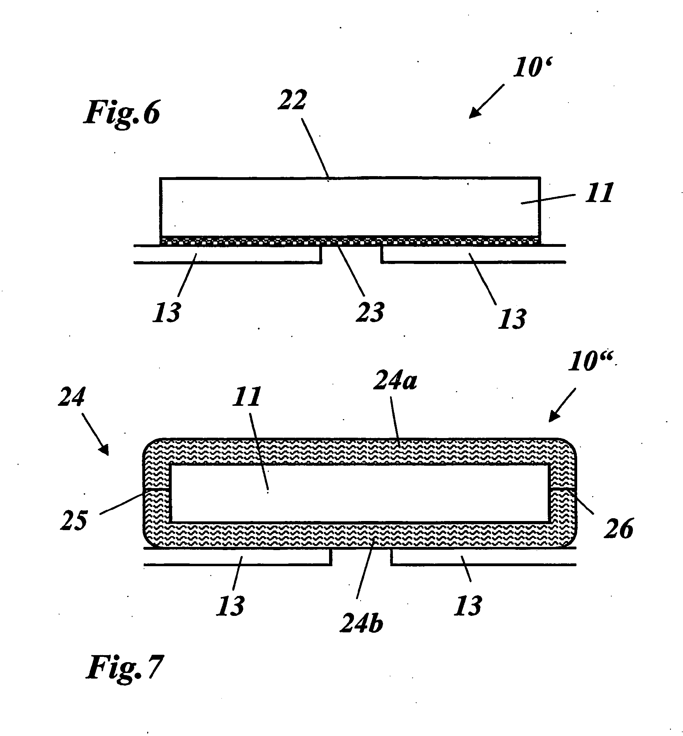

Anstelle des in Fig. 5 gezeigten mehrschichtigen Aufbaus der Mallet-Fingerschiene kann im Rahmen der Erfindung aber auch eine stark vereinfachte Form eingesetzt werden, bei der gemäss Fig. 6 die Mallet-Fingerschiene 10' aus einem einstückigen, gespritzten Kunststoffteil besteht, bei dem auf der Unterseite des Stils 11 direkt beim Spritzen eine Hakenschicht 23 angeformt wird, die später zur Befestigung der beiden Enden eines (wie in Fig. 3 um den geschienten Finger geschlungenen) Befestigungsbandes 13 nach Art eines Klettverschlusses dient. Hierdurch ergeben sich ein besonders einfacher Aufbau und eine besonders einfache Anwendung der Mallet-Fingerschiene 10'. Auf der Oberseite 22 der Mallet-Fingerschiene 10' können zusätzliche Schichten zur Polsterung und/oder Rutschsicherung (z.B. Gewebe mit eingelagerten Silikonkügelchen, etc.) aufgebracht werden, die in Fig. 6 nicht dargestellt sind. Die Hakenschicht 23 kann aber auch auf die Unterseite des Stils 11 aufgeklebt sein.

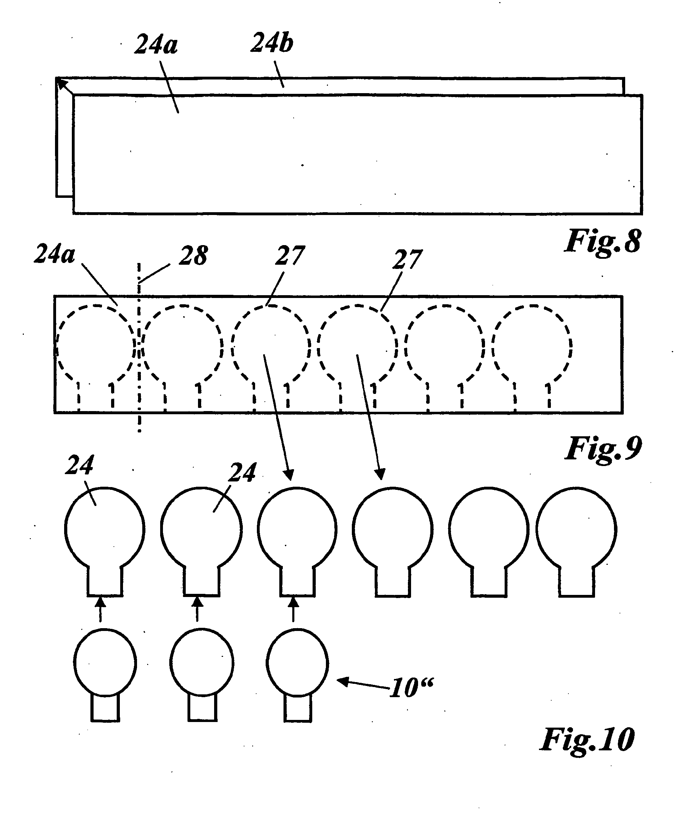

Eine weitere Möglichkeit, im Rahmen der Erfindung eine einfache Mallet-Fingerschiene besonders günstig auch in grossen Stückzahlen herstellen zu können, kann anhand der Fig. 7 und 8-10 erläutert werden: Zunächst werden aus zwei streifenförmigen Lagen 24a, 24b aus unterschiedlichem Material einseitig offene Hüllen 24 hergestellt, in die vorgefertigte Mallet-Fingerschienen 10" in Löffeiform eingeschoben werden können (Fig. 10). Aufbau und Materialwahl der Mallet-Fingerschienen 10" können dabei in weitem Rahmen gewählt werden. Die Hüllen 24 sind so ausgebildet, dass sie auf der dem geschienten Finger zugewandten Oberseite je nach Bedarf die dort notwendigen Funktionen (Polsterung, Aufsaugen von Schweiss; Rutschhemmung bzw. -sicherung, etc.) erfüllen können, und auf der Unterseite zumindest im Bereich des Stils 11 zur lösbaren Befestigung der Enden des Befestigungsbandes 13 dienen.

Die Oberlage 24a und die Unterlage 24b werden gemäss Fig. 8 übereinander gelegt und entlang der in Fig. 9 gestrichelt eingezeichneten Verbindungskonturen 27, die den Randkonturen der Mallet-Fingerschienen 10" entsprechen, untereinander verbunden. Eine Seite bleibt dabei zum Einschieben der Schiene offen. Die Verbindungsnähte zwischen Oberlage 24a und Unterlage 24b (25, 26 in Fig. 7) können durch Nähen, Kleben, Siegeln oder anderweitig erzeugt werden. Nach der Trennung des Lagenverbundes 24a, 24b entlang von Trennlinien 28 und Entfernung des überschüssigen Verbundmaterials ergeben sich die einzelnen Hüllen 24 (Fig. 10). In die elastisch dehnbaren einzelnen Hüllen 24 werden dann die Mallet-Fingerschienen 10" eingeschoben (Fig. 10), und die Hüllen 24 anschliessend verschlossen und/oder mit der Schiene verbunden bzw. verklebt.

Im Querschnitt im Bereich des Stils 11 ergibt sich dann eine Konfiguration, wie sie vereinfacht in Fig. 7 wiedergegeben ist. Die Oberlage 24a kommt beim Schienen mit dem Finger in Berührung. Das Material der Oberlage 24a, beispielsweise ein hautfreundliches Gewebe, sollte entsprechend gewählt sein. Insbesondere können in der Oberlage 24a Mittel zur Sicherung gegen ein Verrutschen des Fingers relativ zur Schiene vorgesehen werden. Hierfür eignen sich insbesondere kleine Kügelchen aus Silikon, die in das Gewebe eingebracht werden und als Rutschhemmer wirken. Die Unterlage 24b kann mit Vorteil zur Befestigung der Enden des Befestigungsbandes 13 herangezogen werden, in dem sie - zumindest im Bereich des Stils 11 - mit hakenartiger Oberfläche nach Art eines Klettverschlusses versehen ist. Hierdurch ergibt sich eine sehr einfache Anbringung der Fingerschiene, sowie ein sehr einfacher und flexibler Aufbau und ein hoher Tragekomfort. Gleichzeitig lässt sich die Fingerschiene kostengünstig in grösseren Serien produzieren. Selbstverständlich können die Ober- und Unterlage 24a,b aber auch bei entsprechender Materialwahl auch weitere zusätzliche Funktionen erfüllen.

### BEZUGSZEICHENLISTE

| | |
|---|---|
| 10,10',10" | Mallet-Flngerschiene |
| 11 | Stiel |
| 12 | Laffe |
| 13 | Befestigungsband |
| 14 | Blech (Al, gewellt) |
| 15,16 | Zwischenschicht (PU-Schaum) |
| 17 | äussere Deckschicht (Flausch) |
| 18 | innere Deckschicht (Futter) |
| 19 | Klettband (doppelseitig) |
| 20,21 | Klebestreifen (doppelseitig) |
| 22 | Oberseite (Stiel) |
| 23 | Hakenschicht |
| 24 | Hülle |
| 24a | Oberlage |
| 24b | Unterlage |
| 25,26 | Verbindungsnaht |
| 27 | Verbindungskontur |
| 28 | Trennlinie |
| 29 | vorderes (distales) Fingergelenk |
| 30 | mittleres Fingergelenk |
| F | Finger |
| F1,..,F3 | Fingerglied |

## Patentansprüche

1. Mallet-Fingerschiene (10, 10', 10") zur Schienung der beiden vorderen Glieder eines Mallet-Fingers (F) in einer Position, bei der das vordere Fingergelenk (29) zwischen dem vorderen (distalen) Fingerglied (F1) und dem mittleren Fingerglied (F2) in einer gestreckten Position gehalten wird, **dadurch gekennzeichnet, dass** die Mallet-Fingerschiene (10, 10', 10") die Form eines Löffels mit einem Stiel (11) und einer Laffe (12) aufweist, dass die Mallet Fingerschiene (10, 10', 10") derart ausgebildet ist, dass der Stiel (11) unter dem mittleren (medialen) Fingerglied (F2) liegt, während das vordere (distale) Fingerglied (F1) in der Laffe (12) zu liegen kommt und dass die Länge des Stiels (11) der Länge des mittleren (medialen) Fingergliedes (F2) derart angepasst ist, dass das mittlere Fingergelenk (30) frei beweglich ist.

2. Mallet-Fingerschiene nach Anspruch 1, **dadurch gekennzeichnet, dass** zumindest auf der Aussenseite des Stiels (11) Mittel (19, 23, 24b) zum lösbaren Befestigen eines Befestigungsbandes (13) vorgesehen sind, welche vorzugsweise Haken nach Art eines Klettverschlusses umfassen.

3. Mallet-Fingerschiene nach Anspruch 1 oder 2, **dadurch gekennzeichnet, dass** an der Mallet-Fingerschiene (10, 10', 10") rutschhemmende Mittel (20, 24a) vorgesehen sind, welche eine relative Verschiebung zwischen Mallet-Fingerschiene (10, 10', 10") und Finger (F) hemmen.

4. Mallet-Fingerschlene nach einem der Ansprüche 1 bis 3, **dadurch gekennzeichnet, dass** die Mallet-Fingerschiene (10, 10', 10") einstückig ausgebildet ist.

5. Mallet-Fingerschiene nach Anspruch 4, **dadurch gekennzeichnet, dass** die Mallet-Fingerschiene (10) aus einem Blech (14) geformt ist, dass als Blech (14) ein Aluminiumblech verwendet wird, dass das Blech (14) mit der Hand plastisch verformbar ist, und dass zur Längsversteifung in das Blech (14) Wellen eingeformt sind, deren Kämme in Schienenlängsrichtung bzw. in Längsrichtung des Fingers (F) verlaufen.

6. Mallet-Fingerschiene nach Anspruch 5, **dadurch gekennzeichnet, dass** das Blech (14) zur Verbesserung der Atmungsaktivität perforiert ist, und dass das Blech (14) auf der Innenseite und/oder der Aussenseite mit wenigstens einer Schicht (15,..,18) bedeckt ist.

7. Mallet-Fingerschiene nach Anspruch 6, **dadurch gekennzeichnet, dass** das Blech (14) auf beiden Seiten mit einer Zwischenschicht (15, 16) aus Kunststoffschaum, insbesondere PU-Schaum, bedeckt ist, und dass auf den Zwischenschichten (15, 16) jeweils eine weitere Deckschicht (17 bzw. 18) angeordnet ist, dass die Zwischenschichten (15, 16) mit dem Blech (14) verklebt sind, und dass die Zwischenschichten (15,16) und die zugehörigen Deckschichten (17, 18) jeweils zusammenlaminiert sind.

8. Mallet-Fingerschiene nach Anspruch7, **dadurch gekennzeichnet, dass** die innere Deckschicht (18) aus einem schweissableitenden Textilmaterial besteht und die äussere Deckschicht (17) aus einem für einen Klettverschluss geeigneten Textilmaterial besteht.

9. Mallet-Fingerschiene nach einem der Ansprüche 1 bis 8, **dadurch gekennzeichnet, dass** zur Befestigung der Mallet-Fingerschiene (10) am Finger (F) ein Befestigungsband (13) vorgesehen ist, welches mit beiden Enden am Stiel (11) lösbar befestigt ist und das darauf liegende mittlere Fingerglied (F2) umschliesst.

10. Mallet-Fingerschiene nach Anspruch 9, **dadurch gekennzeichnet, dass** zur Befestigung des Befestigungsbandes (13) an der Mallet-Fingerschiene (10) auf der Aussenseite des Stiels (11) ein Klettverschluss (19) angeordnet ist, und dass das Befestigungsband (13) zum Zusammenwirken mit dem Klettverschluss (19) auf wenigstens einer Seite mit einem Velours oder Flausch ausgerüstet ist.

11. Mallet-Fingerschiene nach Anspruch 3, **dadurch gekennzeichnet, dass** die rutschhemmenden Mittel einen auf die obere Schicht des Stiels (11) aufgeklebten doppelten Klebestreifen (20) umfassen.

12. Mallet-Fingerschiene nach Anspruch 8, **dadurch gekennzeichnet, dass** die innere Deckschicht (18) zusätzlich mit einer die Vermehrung der Schweissbakterien verhindernden Funktion ausgestattet ist.

13. Mallet-Fingerschiene nach Anspruch 3, **dadurch gekennzeichnet, dass** am Befestigungsband (13) zur Fixierung des Befestigungsbandes (13) relativ zum Finger (F) rutschhemmende Mittel (21) angeordnet sind, welche insbesondere ein doppelseitiges Klebeband (21) umfassen.

14. Mallet-Fingerschiene nach Anspruch 4, **dadurch gekennzeichnet, dass** die Maiiet-Fingerschiene (10') aus einem Kunststoff geformt ist.

15. Mallet-Fingerschiene nach Anspruch 14, **dadurch gekennzeichnet, dass** auf der Aussenseite des Stils (11) eine Hakenschicht (23) als Teil eines Klettverschlusses angebracht ist.

16. Mallet-Fingerschiene nach Anspruch 1, **dadurch gekennzeichnet, dass** die Mallet-Fingerschiene (10") eine Hülle (24) umfasst, dass die Hülle (24) eine Oberlage (24a) und eine Unterlage (24b) aufweist, wobei der zu schienende Finger auf der Oberlage (24a) zu liegen kommt, und die Unterlage (24b) die Mallet-Fingerschiene (10") auf der Unterseite abdeckt, dass die Oberlage (24a) rutschhemmende Eigenschaften hat, und insbesondere Silikon, vorzugsweise in Form von eingebetteten Kügelchen, aufweist, und dass die Unterlage (24b) als Haken enthaltender Teil eines Klettverschlusses ausgebildet ist.

## Claims

1. Mallet finger splint (10, 10', 10") for splinting the two front phalanxes of a mallet finger (F) in a position in which the front finger joint (29) between the front (distal) phalanx (F1) and the middle phalanx (F2) is held in an extended position, **characterized in that** the mallet finger splint (10, 10', 10") has the shape of a spoon with a handle (11) and a shell (12), **in that** the mallet finger splint (10, 10', 10'') is designed in such a way that the handle (11) lies below the middle (medial) phalanx (F2), whilst the front (distal) phalanx (F1) comes to lie in the shell (12), and **in that** the length of the handle (11) is adapted to the length of the middle (medial) phalanx (F2) in such a way that the middle finger joint (30) is freely movable.

2. Mallet finger splint according to Claim 1, **characterized in that** means (19, 23, 24b) for the releasable fastening of a fastening tape (13) are provided at least on the outer side of the handle (11), which means preferably comprise hooks in the manner of a Velcro-type closure.

3. Mallet finger splint according to Claim 1 or 2, **characterized in that** anti-slip means (20, 24a), which prevent a relative movement between mallet finger splint (10, 10', 10") and finger (F), are provided on the mallet finger splint (10, 10', 10'').

4. Mallet finger splint according to one of Claims 1 to 3, **characterized in that** the mallet finger splint (10, 10', 10'') is formed as one piece.

5. Mallet finger splint according to Claim 4, **characterized in that** the mallet finger splint (10) is formed from a sheet metal (14), **in that** an aluminium sheet is used as sheet metal (14), **in that** the sheet metal (14) is plastically deformable by hand, and **in that** corrugations are formed in the sheet metal (14) for longitudinal stiffening, the crests of which corrugations run in the longitudinal direction of the splint or in the longitudinal direction of the finger (F).

6. Mallet finger splint according to Claim 5, **characterized in that** the sheet metal (14) is perforated to improve the breathing activity, and **in that** the sheet metal (14) is covered on the inner side and/or on the outer side with at least one layer (15,..,18).

7. Mallet finger splint according to Claim 6, **characterized in that** the sheet metal (14) is covered on both sides with an intermediate layer (15, 16) of plastic foam, in particular PU foam, **in that** in each case a further cover layer (17 or 18) is arranged on the intermediate layers (15, 16), **in that** the intermediate layers (15, 16) are bonded to the sheet metal (14), and **in that** the intermediate layers (15, 16) and the associated cover layers (17, 18) are in each case laminated together.

8. Mallet finger splint according to Claim 7, **characterized in that** the inner cover layer (18) is made of a textile material that leads away sweat, and the outer cover layer (17) is made of a textile material suitable for a Velcro-type closure.

9. Mallet finger splint according to one of Claims 1 to 8, **characterized in that**, in order to fasten the mallet finger splint (10) to the finger (F), a fastening tape (13) is provided which is releasably fastened with both ends on the handle (11) and which encloses the middle phalanx (F2) lying thereon.

10. Mallet finger splint according to Claim 9, **characterized in that** a Velcro-type closure (19) is arranged on the outer side of the handle (11), for fastening the fastening tape (13) on the mallet finger splint (10), and **in that** the fastening tape (13) is provided with a velour or fleece on at least one side, for engagement with the Velcro-type closure (19).

11. Mallet finger splint according to Claim 3, **characterized in that** the anti-slip means comprise a double adhesive strip (20) bonded onto the upper layer of the handle (11).

12. Mallet finger splint according to Claim 8, **characterized in that** the inner cover layer (18) additionally has a function preventing the formation of sweat bacteria.

13. Mallet finger splint according to Claim 3, **characterized in that** anti-slip means (21), which in particular comprise a double-sided adhesive tape (21), are arranged on the fastening tape (13) for fixing the fastening tape (13) relative to the finger (F).

14. Mallet finger splint according to Claim 4, **characterized in that** the mallet finger splint (10') is formed from a plastic.

15. Mallet finger splint according to Claim 14, **characterized in that** a hook layer (23) is attached as part of a Velcro-type closure to the outer side of the handle (11).

16. Mallet finger splint according to Claim 1, **characterized in that** the mallet finger splint (10'') comprises a sheath (24), **in that** the sheath (24) comprises a top layer (24a) and a bottom layer (24b), the finger to be splinted coming to lie on the top layer (24a), and the bottom layer (24b) covering the mallet finger splint (10") on the underside, **in that** the top layer (24a) has anti-slip properties and in particular comprises silicone, preferably in the form of embedded beads, and **in that** the bottom layer (24b) is designed as the part of a Velcro-type closure containing hooks.

## Revendications

1. Attelle (10, 10', 10'') pour doigt en forme de maillet, pour atteler les deux phalanges avant d'un doigt (F) en maillet en une position dans laquelle l'articulation avant (29) du doigt est maintenue dans une position étendue entre la phalange avant (distale) (F1) du doigt et la phalange centrale (F2) du doigt,
**caractérisée en ce que**
l'attelle (10, 10', 10'') pour doigt en forme de maillet présente la forme d'une cuiller dotée d'un manche (11) et d'une coupelle (12),
**en ce que** l'attelle (10, 10', 10'') pour doigt en forme de maillet est configurée de telle sorte que le manche (11) est situé en dessous de la phalange centrale (médiale) (F2) du doigt tandis que la phalange avant (distale) (F1) du doigt vient se placer dans la coupelle (12) et
**en ce que** la longueur du manche (11) est adaptée à la longueur de la phalange centrale (médiale) (F2) du doigt de telle sorte que l'articulation centrale (30) du doigt reste mobile.

2. Attelle pour doigt en forme de maillet selon la revendication 1, **caractérisée en ce que** des moyens (19, 23, 24b) de fixation libérable d'un ruban de fixation (13) et qui comprennent de préférence des crochets, du type fermeture à crochets, sont prévus au moins sur le côté extérieur du manche (11).

3. Attelle pour doigt en forme de maillet selon les revendications 1 ou 2, **caractérisée en ce qu'**elle présente des moyens (20, 24a) qui bloquent le déplacement relatif entre l'attelle (10, 10', 10'') pour doigt en forme de maillet et le doigt (F) pour empêcher le glissement de l'attelle (10, 10', 10'') pour doigt en forme de maillet.

4. Attelle pour doigt en forme de maillet selon l'une des revendications 1 à 3, **caractérisée en ce que** l'attelle (10, 10', 10'') pour doigt en forme de maillet est configurée d'une seule pièce.

5. Attelle pour doigt en forme de maillet selon la revendication 4, **caractérisée en ce que** l'attelle (10) pour doigt en forme de maillet est formée d'une tôle (14), **en ce que** pour la tôle (14) on utilise une tôle d'aluminium, **en ce que** la tôle (14) est déformable plastiquement à la main et **en ce que** pour rigidifier la tôle (14) dans le sens de la longueur, des ondulations qui s'étendent dans le sens de la longueur de l'attelle et dans le sens de la longueur du doigt (F) y sont formées.

6. Attelle pour doigt en forme de maillet selon la revendication 5, **caractérisée en ce que** pour améliorer l'activité de respiration, le côté intérieur et/ou le côté extérieur de la tôle (14) sont perforés et **en ce que** la tôle (14) est recouverte d'au moins une couche (15, ..., 18).

7. Attelle pour doigt en forme de maillet selon la revendication 6, **caractérisée en ce que** les deux faces de la tôle (14) sont recouvertes par une couche intermédiaire (15, 16) en mousse de matière synthétique et en particulier en mousse de PU, **en ce qu'**une autre couche de recouvrement (17 ou 18) est placée sur les couches intermédiaires (15, 16) respectives, **en ce que** les couches intermédiaires (15, 16) sont collées sur la tôle (14) et **en ce que** les couches intermédiaires (15, 16) sont stratifiées en même temps que les couches de recouvrement (17, 18) associées.

8. Attelle pour doigt en forme de maillet selon la revendication 7, **caractérisée en ce que** la couche intérieure de recouvrement (18) est constituée d'un matériau textile qui évacue la sueur et **en ce que** la couche extérieure de recouvrement (17) est constituée d'un matériau textile qui convient pour une fermeture à crochets.

9. Attelle pour doigt en forme de maillet selon l'une des revendications 1 à 8, **caractérisée en ce qu'**une bande de fixation (13) fixée de manière libérable aux deux extrémités du manche (11) et entourant la phalange centrale (F2) du doigt placée sur le manche est prévue pour fixer l'attelle (10) pour doigt en forme de maillet sur le doigt (F).

10. Attelle pour doigt en forme de maillet selon la revendication 9, **caractérisée en ce qu'**une fermeture à crochets (19) est disposée sur le côté extérieur du manche (11) pour fixer la bande de fixation (13) sur l'attelle (10) pour doigt en forme de maillet et **en ce qu'**au moins un côté de la bande de fixation (13) est doté d'un velours ou d'un tissu à longs poils pour la coopération avec la fermeture à crochets (19).

11. Attelle pour doigt en forme de maillet selon la revendication 3, **caractérisée en ce que** les moyens empêchant le glissement comprennent un ruban adhésif (20) double face collé sur la couche supérieure du manche (11).

12. Attelle pour doigt en forme de maillet selon la revendication 8, **caractérisée en ce que** la couche intérieure de recouvrement (18) est également dotée d'une fonction qui inhibe la prolifération de bactéries due à la sueur.

13. Attelle pour doigt en forme de maillet selon la revendication 3, **caractérisée en ce que** des moyens anti-glissement (21) qui comprennent en particulier un ruban adhésif double face (21) sont disposés sur la bande de fixation (13) pour immobiliser la bande de fixation (13) par rapport au doigt (F).

14. Attelle pour doigt en forme de maillet selon la revendication 4, **caractérisée en ce que** l'attelle (10') pour doigt en forme de maillet est constituée de matière synthétique moulée.

15. Attelle pour doigt en forme de maillet selon la revendication 14, **caractérisée en ce qu'**une couche (23) de crochets qui fait partie d'une fermeture à crochets est placée sur le côté extérieur du manche (11).

16. Attelle pour doigt en forme de maillet selon la revendication 1, **caractérisée en ce que** l'attelle (10'') pour doigt en forme de maillet comprend une enveloppe (24), **en ce que** l'enveloppe (24) présente une couche supérieure (24a) et une couche inférieure (24b), le doigt à atteler venant se placer sur la couche supérieure (24a), la couche inférieure (24b) recouvrant le côté inférieur de l'attelle (10'') pour doigt en forme de maillet, **en ce que** la couche supérieure (24a) a des propriétés anti-glissement et présente en particulier du silicone, de préférence sous la forme de petites sphères incorporées, et **en ce que** la couche inférieure (24b) est configurée comme partie contenant les crochets d'une fermeture à crochets.
